# EUROPÄISCHE PATENTSCHRIFT

(11) **EP 1 194 110 B1**
(45) Veröffentlichungstag und Bekanntmachung des Hinweises auf die Patenterteilung: **29.12.2004**
(21) Anmeldenummer: 00938806.7
(22) Anmeldetag: 21.06.2000
(51) Int. Cl.: A61K 6/083

(54) **POLYMERISIERBARE DENTALMASSE**
POLYMERIZABLE DENTAL SUBSTANCE
MATIERE DENTAIRE POLYMERISABLE

(30) Priorität: 21.06.1999 DE 19928238
(43) Veröffentlichungstag der Anmeldung: 10.04.2002
(73) Patentinhaber: 3M ESPE AG, 82229 Seefeld (DE)
(72) Erfinder: SOGLOWEK, Wolfgang, D-86911 Diessen-Obermühlhausen (DE); O'CONNELL, Keith, Maidenhead, Berkshire SL6 4UZ (GB)
(86) Internationale Anmeldenummer: PCT/EP2000/005737
(87) Internationale Veröffentlichungsnummer: WO 2000/078271

(56) Entgegenhaltungen:
- EP-A- 0 059 451
- EP-A- 0 374 824
- EP-A- 0 508 095
- EP-A- 0 732 098
- WO-A-96/19179

## Beschreibung

Die vorliegende Erfindung betrifft polymerisierbare Dentalmassen, die
(a) 10 bis 98,999 Gew. % mindestens eines bi- oder höherfunktionellen ethylenisch ungesättigten Monomers,
(b) 0 bis 88,999 Gew. % mindestens eines monofunktionellen ethylenisch ungesättigten Monomers,
(c) 0 bis 5 Gew.% eines Beschleunigers,
(d) 0,001 bis 5 Gew.% eines Redoxinitiatorsystems; das die radikalische Polymerisation auslösen kann,
(e) 0 bis 88,999 Gew. % Füllstoffe, Thixotropie-Hilfsmittel, Verzögerer und anderer Hilfsstoffe und
(f) 1 bis 30 Gew. % eines üblichen Weichmachers
enthalten.

Polymerisierbare Dentalmassen sind insbesondere geeignet als Füllungsmaterialien, Stumpfaufbaumaterialien, Befestigungszemente, provisorische Kronen- und Brückenmaterialien, zahntechnische Werkstoffe, Modellmaterial oder zur Herstellung von Inlays, Onlays, Verblendschalen und provisorischen Kronen und Brücken.

Je nach Anwendungszweck hat man es hierbei mit dünnfließenden bis zähplastischen Massen zu tun, die mit organischen oder anorganischen Füllstoffen versehen sein können, und während der Polymerisation aushärten.

Als Monomere der polymerisierbaren Dentalmassen werden vor allem ethylenisch ungesättigte Verbindungen, wie Acrylsäure- und/oder Methacrylsäureester, verwendet.

Bei den provisorischen Kronen- und Brückenmaterialien handelt es sich üblicherweise um relativ niedrig gefüllte Systeme, die ca. 10 bis 70 Gew. % anorganischen Füllstoff enthalten. Die verwendeten Füllstoffe haben eine mittlere Korngröße von 1 bis 15 µm. Zusätzlich werden aber auch wesentlich feinere Füllstoffe im Bereich von 0,02 bis 0,05 µm mit den oben genannten Füllstoffen eingesetzt, um die Massen ausreichend plastisch und thixotrop zu machen. Besonders bewährt hat sich auch die Verwendung von organischen Füllstoffen, wie z.B. Polymethylmethacrylat.

Bei der Anwendung dieser Materialien werden die Monomere kurz vor der Verarbeitung mit geeigneten Initiatorsystemen vermischt, wobei eine pastöse Masse entsteht, die durch radikalische Polymerisation aushärtet. Dabei können die Ausgangs-Komponenten, die unter anderem Monomer und Initiatorsystem aufweisen, ebenfalls in Form von räumlich voneinander getrennten Pasten oder auch als Pulver-Flüssigkeits-System vorliegen.

Als Ausloser der radikalischen Polymerisation werden verschiedene Initiatorsysteme eingesetzt. Dabei ist es erforderlich, daß nach Start der Polymerisation die Verarbeitungszeit bis zum Aushärten des Materials lange genug ist, um dem Zahnarzt genügend Zeit für eine Anpassung des Materials und zur Verarbeitung zur Verfügung zu stellen. Gleichzeitig soll aber die Abbindephase vom Beginn der Gelierung bis zu einer weitgehenden Aushärtung des Materials möglichst kurz sein, da während dieser Phase eine Bearbeitung nicht möglich ist und die Wartezeit für den Zahnarzt und den Patienten möglichst kurz sein sollte.

Ein bereits lange bekanntes Initiatorsystem besteht aus einer Amin- und einer Peroxidkomponente, wie z.B. in der Patentschrift DE-C- 975 072 beschrieben. Die Polymerisation wird hierbei durch die Peroxidverbindung gestartet. Als Beschleuniger der Polymerisation wird beispielsweise ein tertiäres Amin eingesetzt. Ein weiteres solches System wird auch von Albert Groß in "Quintessenz der Zahntechnik", 1977, 7, Referat Nr. 293, beschrieben. Dort beschleunigen sekundäre oder tertiäre Amine den Zerfall der Peroxidkomponente, die die Polymerisation des Materials auslöst. Üblicherweise wird die Amin-Komponente dabei in eine Paste, die sogenannte Basispaste, eingearbeitet. Diese Basispaste enthält auch die zur Polymerisation vorgesehenen Monomere. Die Peroxid-Komponente wird in eine weitere Paste, die sogenannte Katalysatorpaste, eingearbeitet. Die räumliche Trennung der beiden Initiatorkomponenten ist erforderlich, um eine vorzeitige Aushärtung der Monomeranteile zu vermeiden. Auch in der deutschen Patentschrift DE-C-955 633 wird ein ähnliches Initiatorsystem für die Polymerisation von ungesättigten Kohlenwasserstoffen beschrieben, das Schwermetalle sowie eine Amin- und eine Sulfonkomponente enthält. Auch in der europäischen Patentschrift EP-B-0 374 824 wird ein Initiatorsystem mit einer organischen Peroxidverbindung und einem tertiären aromatischen Amin als Aktivator (Beschleuniger) genannt.

Nachteilig an den genannten Materialien ist, daß die für eine günstige Abbindephase geeigneten Amine zu Verfärbungen neigen. Diese gelb-braunen Farbveränderungen sind im dentalen Bereich aber nicht akzeptabel. Außerdem sind tertiäre aromatische Amine aufgrund ihrer Gesundheitsgefährdung nur bedingt einsetzbar. Weiterhin problematisch ist der Temperaturanstieg bei der Polymerisation dieser Systeme aufgrund der exothermen Reaktionsprozesse. Eine zu hohe Wärmeentwicklung kann zu Pulpaschädigungen des Patienten führen.

Eine günstigere Temperaturentwicklung und auch bessere Farbstabilität weisen die Initiatorsysteme auf, die in der deutschen Patenschirft DE-C-14-95 520 beschrieben werden. Die Zusammensetzung aus der DE-C-14 95 520 polymerisiert bei niedriger Temperatur in kurzer Zeit und ohne Anwendung von externer Energie. Die beschriebenen Systeme enthalten-Barbitursäurederivate bzw. Malonylsulfamide, organische Peroxide, ionogen gebundenes Halogen und/oder eine Schwermetallverbindung. Auch die europäische Patentschrift EP-B-0- 374 824 beschreibt ein derartiges Initiatorsystem aus Barbitursäurederivat, Peroxid, Schwermetallverbindung und ionogenem Halogen. Bei diesen Initiatorsystemen können Barbitursäurederivate bzw. Malonylsulfamide und Peroxide nicht gemeinsam gelagert werden. Ferner müssen auch beide genannten Bestandteile des Initiatorsystems von den Monomeren getrennt aufbewahrt werden. Es ist also zur Bereitstellung von polymerisierbaren Dentalmassen, die Monomere, Barbitursäurederivate bzw. Malonylsulfamide, organische Peroxide, ionogen gebundenes Halogen und/oder eine Schwermetallverbindung enthalten, eine Lagerung in drei räumlich voneinander getrennten Pasten notwendig.

Dies führt zu einer relativ aufwendigen Handhabung der Systeme. So sind für eine automatische Anmischung dreikomponentige Systeme nicht geeignet. Daher müssen diese herkömmlichen Dentalmassen aus drei Komponenten von Hand angemischt werden, wobei Luft eingearbeitet wird und die Dosierung der Einzelkomponeneten nicht so genau erfolgen kann. Die Einarbeitung von Luft ist vor allem deshalb zu vermeiden, da durch eingearbeitete Luftblasen Fehlstellen in dem ausgehärteten Material entstehen. Dadurch erhöht sich die Bruchempfindlichkeit und es resultiert eine schlechte Oberflächenbeschaffenheit. Unterschiedliche Dosierungen führen zu veränderten Abbindezeiten, verschlechterten mechanischen Eigenschaften und einer ungenauen Farbgebung. Ferner ist die Handanmischung zeitaufwendiger als eine automatische Mischung.

Alternativ besteht die Möglichkeit, wie z.B. in der JP-A-02245080 beschrieben, Barbitursäurederivate und organische Peroxide in Form eines Pulvers zur Verfügung zu stellen.

Derartige Pulver-Flüssigkeits-Systeme werden auch in der DE-A-197 42 980 und der US-A-5,688,883 beschrieben. Die Peroxidkomponente ist in einer Lösung enthalten, während die Pulverkomponente ein Barbitursäurederivat aufweist.

In der deutschen Patentschrift DE-C-37 25 502 wird ein Zweikomponentensystem mit den genannten Bestandteilen beschrieben. Hierbei handelt es sich jedoch um ein Pulver-Flüssigkeits-System, bei dem Barbitursäurederivate bzw. Malonylsufamide und Peroxide als Pulver vorliegen und eine ausreichende Lagerstabilität gegeben ist, weil die beiden Bestandteile in pulverförmigem Zustand nicht nennenswert miteinander -reagieren, Nachteilig an -einem solchen Pulver-Flüssigkeits-System ist die umständliche Handhabung. Z.B. sind diese Systeme aufgrund des Pulveranteils nicht für einen Einsatz in handelsüblichen Mischgeräten des Dentalbereichs, die für Paste-Paste-Systeme ausgelegt sind, -einsetzbar. Außerdem wird für die Anmischung einer polymerisierbaren Dentalmasse aus Pulver und Flüssigkeit mehr -Zeit benötigt als für die Anmischung von Pasten und es besteht die Gefahr, -daß Luft eingearbeitet wird mit den oben beschriebenen Nachteilen.

Zweikomponentige, automatisch anmischbare Paste-Paste-Systeme aus den Bestandteilen, die in den genannten Druckschriften DE-C-37 25 502, DE-C-14 95 520 und EP-B-0 374 824 aufgeführt werden, waren bisher nicht mit ausreichender Lagerstabilität herstellbar.

Es ist daher die Aufgabe der vorliegenden Erfindung, eine polymerisierbare Dentalmasse zur Verfügung zu stellen, deren Initiatorsystem (i) Barbitursäurederivate und/oder Malonylsulfamide und (ii) organische Peroxide sowie gegebenenfalls ionogene Halogene und Schwermetallverbindungen enthält, deren Bestandteile in zwei Komponenten in Form von räumlich voneinander getrennten Pasten vorliegen können, und die dabei eine ausreichende Lagerstabilität aufweist. In der Regel sollten solche Massen mindestens ein Jahr lagerfähig sein, um nach dem Vertrieb beim Zahnarzt noch angemessene Restlaufzeiten und Aufbrauchfristen zu haben.

Diese Aufgabe wird gelöst durch die Bereitstellung einer Dentalmasse, die
(a) 10 bis 98,999 Gew. % mindestens eines bi- oder höherfunktionellen ethylenisch ungesättigten Monomeren,
(b) 0 bis 88,999 Gew. % mindestens eines monofunktionellen ethylenisch ungesättigten Monomeren,
(c) 0 bis 5 Gew. % eines Beschleunigers
(d) 0,001 bis 5 Gew. % eines Redoxinitiatorsystems, das die radikalische Polymerisation auslösen kann,
(e) 0 bis 88,999 Gew. % Füllstoffe, Thixotropie-Hilfsmittel, Verzögerer und anderer Hilfsstoffe und
(f) 1 bis 30 Gew. % eines üblichen Weichmachers
enthält und dadurch gekennzeichnet ist, daß das Redoxinitiatorsystem (i) ein Barbitursäurederivat und/oder ein Malonylsulfamid und (ii) ein organisches Peroxid, ausgewählt aus der Gruppe der ein- oder mehrfunktionellen Carbonsäureperoxyester, umfasst und daß die Bestandteile (a) bis (f) in zwei räumlich voneinander getrennten Pasten vorliengen.

Überraschenderweise wurde nämlich festgestellt, daß aufgrund des speziellen Einsatzes eines organischen Peroxids aus der Gruppe der ein oder mehrfunktionellen Carbonsäureperoxyester, die Bestandteile der polymerisierbaren Dentalmasse (a) bis (f) in zwei räumlich voneinander getrennten Pasten vorliegen können. Bei den aus dem Stand der Technik bekannten Zubereitungen war eine solche Darreichung nicht möglich, da, wie bereits oben beschrieben, in derjenigen Komponente, die gleichzeitig (i) ein Barbitursäurederivat und/oder ein Malonylsulfamid und (ii) ein übliches organisches Peroxid aufwies, innerhalb kurzer Lagerzeit diese beiden Bestandteile miteinander reagierten und dadurch eine Abbindung nach Zumischen der monomerhaltigen Komponente nicht mehr innerhalb einer angemessenen Zeitspanne erfolgte.

Bei Verwendung der erfindungsgemäßen organischen Peroxide ist wider Erwarten eine ausreichende Lagerstabilität derjenigen Komponente, die gleichzeitig (i) ein Barbitursäurederivat und/oder ein Malonylsulfamid und (ii) ein organisches Peroxid aufweist, gefunden worden, so daß eine Bereitstellung in Form von zwei räumlich voneinander getrennten Pasten möglich wird.

Die beiden räumlich voneinander getrennten Pasten werden als Basis- und Katalysatorpaste bereitgestellt, wobei die Basispaste die Bestandteile (a), (b) und (c) und die Katalysatorpaste die Bestandteile (d) und (f) aufweist. Ferner enthält Basispaste zusätzlich den Bestandteil (e) und/oder (f), die Katalysatorpaste zusätzlich den Bestandteil (e).

Unter dem Begriff "monofunktionelle" bzw. "bi- oder höherfunktionelle ethylenisch ungesättigte Monomere" im Sinne der vorliegenden Erfindung sind auch solche polymerisierbaren Verbindungen zu verstehen, die ein oligomeres bzw. polymeres Grundgerüst besitzen und mindestens eine ethylenisch ungesättigte Gruppe tragen. Diese ethylenisch ungesättigte Gruppe kann beispielsweise als Acrylat- und/oder Methacrylatgruppe vorliegen, die an das Grundgerüst kovalent gebunden ist. Das polymere Grundgerüst kann z.B. ein Polyethylenoxid, ein Polyester, ein Polyurethan, ein Polycarbonat, ein Polyalkohol, ein Polystyrol oder eine polymerisierte ethylenisch ungesättigte Verbindung sein.

Besonders bevorzugt als monofunktionelle bzw. bi- oder höherfunktionelle ethylenisch ungesättigte Monomere gemäß Bestandteil (a) oder (b) sind Methacrylat- und Acrylatmonomere, wie z.B. Methyl(meth)acrylat, n- oder i-Propyl(meth)acrylat, n-, i- oder tert.-Butyl(meth)acrylat und 2-Hydroxyl(meth)acrylat, 2-(Meth)acryloxy-tetrahydrofuran, 2-(((Alkylamino)-carbonyl)oxy)ethyl-(meth)acrylate; Di(meth)acrylate des Propandiols, Butandiols, Hexandiols, Octandiols, Nonandiols, Decandiols und Eicosandiols; Di(meth)acrylate des Ethylenglycols, der Polyethylenglycole, der Pölypropylenglycole; Di(meth)acrylate des ethoxylierten Bisphenol A, -z.B. 2,2'-Bis(4-(meth)acryloxy-tetraethoxyphenyl)propane; Urethan(meth)acrylate; (Meth)acryl-amide.

Ferner können als Monomere der Bestandteile (a) und (b) Ester der α-Cyanoacrylsäure, Crotonsäure, Zimtsäure, Sorbinsäure; Vinylether, wie z.B. Butylvinylether; Mono-N-Vinyl-Verbindungen, wie N-Vinylpyrrolidon, verwendet werden.

Weiterhin verwendbar sind die in der europäischen Offenlegungsschrift EP-A-0 235 826 genannten Methacrylsäureester, wie z.B. Triglykolsäure-bis[3[4]-methacryloxymethyl-8(9)-tricyclo-[5.2.1.0^{2,6}]-decylmethylester

Insbesondere geeignet sind 2,2-Bis-4(3-methacryloxy-2-hydroxypropoxy)phenylpropan (bis-GMA), 2,2-Bis-4(3-methacryloxy-propoxy)phenylpropan, Triethylenglykoldimethacrylat (TEGDMA), 7,7,9-Trimethyl-4,13-dioxo-3,14,-dioxa-5,12-diazahexadecan-1,16-dioxydimethacrylat (UDMA) und Di(meth)acrylate des Bishydroxymethyltricyclo(5.2.1.0^{2,6})-decans

Diese ethylenisch ungesättigten Monomere können in den offenbarten Dentalmassen entweder alleine oder in Kombination mit weiteren ethylenisch ungesättigten Monomeren eingesetzt werden.

Erfindungsgemäß werden die bi- oder höherfunktionellen ethylenisch ungesättigten Monomere in einer Konzentration von 10 bis 98,999 Gew. %, vorzugsweise von 30 bis 80 Gew. %, jeweils bezogen auf die Gesamtmasse der Bestandteile (a) bis (f), verwendet. Besonders bevorzugt ist ein Einsatz von 45 bis 70 Gew. % des Bestandteils (a) in der polymerisierbaren Dentalmasse.

Der Bestandteil (b) wird in einer Konzentration von 0 bis 88,999 Gew. %, bezogen auf die Gesamtmasse der Bestandteile (a) bis (f), eingesetzt. Insbesondere eignet sich eine Konzentration an monofunktionellen ethylenisch ungesättigten Monomeren gemäß Bestandteil (b) von 0 bis 58,99 Gew. %, bevorzugt von 0 bis 33,99 Gew. %.

Als Beschleuniger gemäß Bestandteil (c) sind Schwermetallverbindungen, insbesondere Metalle der Eisen- oder der Kupfergruppe, bevorzugt Kupfer geeignet. Das Schwermetall wird geeigneterweise in Form löslicher organischer Verbindungen eingesetzt. Außerdem können ionogen gebundene Halogene oder Pseudohalogene; z.B. Cl- enthaltende Verbindungen, bevorzugt in Form von löslichen Salzen, insbesondere organische Ammoniumchloride oder Hydrochloride, als Beschleuniger zugesetzt werden. Diese Verbindungen sind in der polymerisierbaren Dentalmasse in einer Konzentration von 0 bis 5 Gew.-%, vorzugsweise von 0 bis 3 Gew.%; besonders bevorzugt von 0,05 bis 2 Gew. %, enthalten. Es kann auch ein Gemisch von mehreren Beschleunigern verwendet werden:

Ferner kann die erfindungsgemäße polymerisierbare Dentalmasse als Bestandteil (e) 0 bis 88,999 Gew. %, insbesondere 10 bis 68,99 Gew. % und besonders bevorzugt 20 bis 53,9 Gew. % übliche Füllstoffe für Dentalwerkstoffe, wie beispielsweise Glas- und Quarzpulver, Kieselgele, pyrogene hochdisperse Kieselsäuren oder schwer lösliche Fluoride sowie Mischungen dieser Komponenten enthalten. Diese Füllstoffe können durch geeignete Zusätze, wie beispielsweise barium- oder strontiumhaltige Gläser, röntgenopak sein. Als Thixotropie-Hilfsmittel sind z.B. pyrogene hochdisperse Kieselsäuren geeignet. Weitere Hilfsstoffe sind beispielsweise Farbstoffe, Pigmente, Fließverbesserer, polymere Verdickungsmittel oder Stabilisatoren. Zur Erhöhung der Flexibilität der Dentalmasse können auch lösliche organische Polymerisate, wie z.B. Polyvinylacetat sowie dessen Copolymere, zugesetzt werden.

Auch Christobalit, Calciumsilikat, Zirkoniumsilikat, Montmorillonite, wie Bentonite, Zheolite, einschließlich der Molekularsiebe, wie Natriumaluminiumsilikat, Metalloxidpulver, wie Aluminium- oder Zinkoxide bzw. deren Mischoxide, Bariumsulfat, Yttriumfluorid, Calciumcarbonat, Gips und Kunststoffpulver sind als Füllstoffe für die erfindungsgemäße Dentalmasse geeignet.

Die genannten Füllstoffe können auch durch z.B. eine Behandlung mit Organosilanen bzw. - siloxanen oder durch die Veretherung von Hydroxylgruppen zu Alkoxygruppen hydrophobiert sein.

Als Verzögerer sind die in der europäischen Patentschrift EP-B-0 374 824 beschriebenen Verbindungen geeignet.

Das erfindungsgemäß einzusetzende Redoxinitiatorsystem besteht aus (i) einem Barbitursäurederivat und/oder einem Malonylsulfamid und (ii) einem organischen Peroxid, ausgewählt aus der Gruppe der ein- oder mehrfunktionellen Carbonsäureperoxyester. Als Barbitursäurederivate können beispielsweise eingesetzt werden: 1,3,5- Trimethylbarbitursäure, 1,3,5-Triethylbarbitursaüre, 1,3-Dimethyl-5-ethylbarbitursäure, 1,5-Dimethylbarbitursäure, 1-Methyl-5-ethylbarbitursäure, 1-Methyl-5-propylbarbitursäure, 5-Ethylbarbitursäure, 5-Propylbarbitursäure, 5-Butylbarbitursäure, 1-Benzyl-5-phenylbarbitursäure, 1-Cyclohexyl-5-ethylbarbitursäure und die in der deutschen Offenlegungsschrift DE-A-42 19 700 genannten Thiobarbitursäuren.

Gut geeignet sind auch die in der deutschen Patentschrift DE-C-14 95 520 beschriebenen Barbitursäuren und Barbitursäurederivate, sowie die in der europäischen Patentschrift EP-B-0 059 451 genannten Malonylsulfamide. Bevorzugte Malonylsulfamide sind 2,6-Dimethyl-4-isobutylmalonylsulfamid, 2,6-Diisobutyl-4-propylmalonylsulfamid, 2,6-Dibutyl-4-propylmalonylsulfamid, 2,6-Dimethyl-4-ethylmalonylsulfamid oder 2,6-Dioctyl-4-isobutylmalonylsulfamid.

Als organische Peroxide weist das erfindungsgmäße Redoxinitiatorsystem ein- oder mehrfunktionelle Carbonsäureperoxyester auf. Unter den mehrfunktionellen Carbonsäureperoxyestern im Sinne der vorliegenden Anmeldung sind auch Kohlensäureperoxyester zu verstehen.

Geeignet sind beispielsweise Kohlensäure-diisopropyl-peroxydiester, Neodecansäure-tertiärbutylperoxyester, Neodecansäure-tertiäramyl-peroxyester, Maleinsäure-tertiärbutyl-monoperoxyester, Benzoesäure-tertiärbutyl-peroxyester, 2-Ethylhexansäure-tertiärbutyl-peroxyester, 2-Ethylhexansäure-tertiäramyl-peroxyester, Kohlensäure-monoisopropylester-monotertiärbutyl-peroxyester, Kohlensäure-dicyclohexyl-peroxyester, Kohlensäure-dimyristyl-peroxyester, Kohlensäuredicetyl-peroxyester, Kohlensäure-di(2-ethylhexyl)-peroxyester, Kohlensäure-tertiärbutyl-peroxy-(2-ethylhexyl)ester oder 3,5,5-Trimethylhexansäure-tertiärbutyl-peroxyester, Benzoesäuretertiärarnyl-peroxyester, Essigsäure-tertiärbutyl-peroxyester, Kohlensäure-di(4-tertiärbutylcyclohexyl)-peroxyester, Neodecansäure-cumol-peroxyester, Pivalinsäure-tertiäramyl-peroxyester und Pivalinsäure-tertiärbutyl-peroxyester.

Insbesondere können Kohlensäure-tertiärbutyl-peroxy-(2-ethylhexyl)ester oder 3,5,5-Trimethylhexansäure-tertiärbutyl-peroxyester als erfindungsgemäße organische Peroxide verwendet werden.

Die erfindungsgemäßen Dentalmassen weisen das Redoxinitiatorsystem in einer Konzentration von 0,001 bis 5 Gew.-%, vorzugsweise in einer Konzentration von 0,01 bis 3 Gew. %, bezogen auf die Gesamtmasse der Bestandteile (a) bis (f), auf. Besonders bevorzugt ist eine Konzentration -an Initiatorsystem von 0,05 bis 2 Gew. %.

Als Bestandteil (f) enthält die erfindungsgemäße polymerisierbare Dentalmasse 1 bis 30 Gew. %, vorzugsweise 1 bis 20 Gew. %, insbesondere 1 bis 15 Gew. %, -eines üblichen Weichmachers bzw. eines Gemischs von üblichen Weichmachern. Dies sind beispielweise Polyethylenglycolderivate, Polypropylenglycole, niedermolekulare Polyester, Dibutyl-, Dioctyl-, Dinonyl-, Diphenylphthalat, Di(iso-nonyladipat), Tricresylphosphat und Siliconöle.

Für die nachfolgenden Beispiele wurden die erfindungsgemäßen Dentalmassen als zweikomponentige pastenförmige Systeme in Form einer Basis- und einer Katalysatorpaste hergestellt. Die Basispaste enthielt die Bestandteile (a), (b), (c) und (e) in den aus der Tabelle 1 ersichtlichen Mengen in Gew.%. Bestandteil (f) kann wahlweise ebenfalls zugesetzt werden. Die Katalysatorpaste enthielt die Bestandteile (d), (e) und (f), wobei (e) nicht obligatorisch enthalten sein muß. Zur Herstellung der beiden Pasten wurden die jeweiligen Bestandteile der Basis- und der Katalysatorpaste mit einem Dreiarmkneter unter Vakuum zu homogenen Pasten verknetet.

Die Katalysatorpasten 1, 2 und 3 enthielten übliche, aus dem Stand der Technik bekannte Peroxide. Diese Pasten dienten zur Herstellung von Vergleichsversuchen. Die Katalysatorpasten 4 und 5 wiesen die erfindungsgemäßen Peroxide auf.

Für die nachfolgenden Untersuchungen wurde ein Mischungsverhältnis von Basis- zu Katalysatorpaste von 10:1 gewählt. Selbstverständlich sind die erfindungsgemäßen zweikomponentigen Systeme auch mit anderen Mischungsverhältnissen herstellbar.

**Tabelle 1**

| **BASISPASTE** | Glaspulver | 34 Gew.% |
|---|---|---|
| | mikrofeine Kieselsäure | 8 Gew.% |
| | Bis-(1-phenylpentan-1,3-dionato)-kupfer(II) | 0,0013 Gew.% |
| | (β-Phenylethyl)-dibutyl-ammonium-chlorid | 0,36 Gew.% |
| | 2,2-Bis-{4-[oligo(ethoxy)]phenyl}-propan-dimethacrylat | auf 100 Gew.% aufgefüllt |
| **KATALYSATOR-PASTE 1** | Glaspulver | 34 Gew.% |
| | mikrofeine Kieselsäure | 8 Gew.% |
| | 1-Benzyl-5-phenylbarbitursäure | 0,6 Gew.% |
| | 2,2-Bis-4-(2-hydroxyethoxyphenyl)-propan-bis-acetat | auf 100 Gew.% aufgefüllt |
| | Dibenzoylperoxid | 3,9 Gew:% |
| **KATALYSATOR- PASTE 2** | Glaspulver | 34 Gew.% |
| | mikrofeine Kieselsäure | 8 Gew.% |
| | 1-Benzyl-5-phenylbarbitursäure | 0,6 Gew.% |
| | 2,2-Bis-4-(2-hydroxyethoxyphenyl)-propan-bis-acetat | auf 100 Gew.% aufgefüllt |
| | Dilauroylperoxid | 8,0 Gew.% |
| **KATALYSATOR- PASTE 3** | Glaspulver | 34 Gew.% |
| | mikrofeine Kieselsäure | 8 Gew.% |
| | 1-Benzyl-5-phenylbarbitursäure | 0,3 Gew.% |
| | 2,2-Bis-4-(2-hydroxyethoxyphenyl)-propan-bis-acetat | auf 100 Gew.% aufgefüllt |
| | Cumol-hydroperoxid | 0,48 Gew.% |
| **KATALYSATOR- PASTE 4** | Glaspulver | 34 Gew.% |
| | mikrofeine Kieselsäure | 8 Gew.% |
| | 1-Benzyl-5-phenylbarbitursäure | 0,6 Gew.% |
| | 2,2-Bis-4-(2-hydroxyethoxyphenyl)-propan-bis-acetat | auf 100 Gew.% aufgefüllt |
| | Kohlensäure-tertiärbutylperoxy-(2-ethylhexyl)ester | 0,6 Gew.% |
| **KATALYSATOR- PASTE 5** | Glaspulver | 34 Gew.% |
| | mikrofeine Kieselsäure | 8 Gew.%. |
| | 1-Benzyl-5-phenylbarbitursäure | 0,6 Gew.% |
| | 2,2-Bis-4-(2-hydroxyethoxyphenyl)-propan-bis-acetat | auf 100 Gew.% aufgefüllt |
| | 3,5,5-Trimethylhexansäure-tertiärbutylperoxyester | 0,6 Gew.% |

Die verschiedenen Katalysatorpasten der erfindungsgemäßen Dentalmassen wurden über längere Zeiträume gelagert und zu verschiedenen Lagerzeiten die Abbindezeiten der aus den beiden Komponenten angemischten Dentalmassen bestimmt.

Die Abbindezeiten wurden nach maximal 3 Tagen Lagerung bei Raumtemperatur bestimmt. Dieser Wert ist in der Tabelle 2 als Startwert ausgewiesen. Anschließend wurde die Katalysatorpaste in einem Wärmeschrank bei 50°C gelagert und die Abbindezeit nach den angegebenen Zeitabständen kontrolliert (siehe Tabelle 2). Die Basispaste wurde bei Raumtemperatur gelagert. Die Wärmelagerung bei 50 °C ist ein allgemein anerkannter Test unter Streßbedingungen, der aufgrund der Ergebnisse, die bei erhöhten Temperaturen erhalten werden, Rückschlüsse auf die Stabilität bei einer Lagerung, unter normalen Temperaturen erlaubt. Dabei sind diese Temperaturen aber durchaus auch realistisch, da die beschriebenen Dentalmassen z.B. während des Transports derartigen Temperaturen ausgesetzt sein können.

Die Abbindezeiten wurden folgendermaßen bestimmt:
Die Basispaste (1,00 g) und die entsprechende Katalysatorpasten 1-5 (0,10 g) wurden auf einem Mischblock eingewogen und homogen vermischt. Die Abbindezeit der gemischten Paste wurde mittels eines Curometers (Fa. Wallace-Shawbury, England) bestimmt.

Die Ergebnisse der gemessenen Abbindezeiten zeigt die Tabelle 2, wobei der Ausdruck >20min bedeutet, daß innerhalb von 20 min keine vollständige Abbindung beobachtet wurde und deshalb der jeweilige Versuch abgebrochen wurde.

**Tabelle 2**

| | **Start** | **1 Tag (50°C)** | **3 Tage (50°C)** | **1 Woche (50°C)** | **2 Wochen (50°C)** | **3 Wochen (50°C)** |
|---|---|---|---|---|---|---|
| **KATALYSATOR- PASTE 1** | | | | | | |
| Abbindezeit [min'sec] | 2'55 | > 20'00 | > 20'00 | > 20'00 | > 20'00 | > 20'00 |

| **KATALYSATOR-PASTE 2** | | | | | | |
|---|---|---|---|---|---|---|
| Abbindezeit [min'sec] | 2'35 | | > 20'00 | > 20'00 | > 20'00 | > 20'00 |

| **KATALYSATOR-PASTE 3** | | | | | | |
|---|---|---|---|---|---|---|
| Abbindezeit[min sec] | 3'45 | | >20'00 | >20'00 | >20'00 | > 20'00 |

| **KATALYSATOR- PASTE 4** | | | | | | |
|---|---|---|---|---|---|---|
| Abbindezeit [min sec] | 2'00 | 2'00 | 2'05 | 2'25 | 2'25 | 2'35 |

| **KATALYSATOR PASTE 5** | | | | | | |
|---|---|---|---|---|---|---|
| Abbindezeit [min'sec] | 2'05 | 2'00 | 1'55 | 1'55 | 2'00 | 2'10 |

## Patentansprüche

1. Polymerisierbare Dentalmasse, enthaltend
(a) 10. bis 98,949 Gew.-% mindestens eines bi- oder höherfunktionellen ethylenisch ungesättigten Monomers,
(b) 0 bis 88, 999 Gew.-% mindestens eines monofunktionellen ethylenisch ungesättigten Monomers,
(c) 0,05 bis 5 Gew.-% eines Beschleunigers,
(d) 0,001 bis 5 Gew.-% eines Redoxinitiatorsystems, das die radikalische Polymerisation auslösen kann, und
(e) 0 bis 88,999 Gew.-% Füllstoffe, Thixotropie-Hilfsmittel, Verzögerer und anderer Hilfsstoffe,
(f) 1 bis 30 Gew.-% eines üblichen Weichmachers, **dadurch gekennzeichnet, daß** das Redoxinitiatorsystem (i) ein Barbitursäurederivat und/oder ein Malonylsulfamid und (ii) ein organisches Peroxid, ausgewählt aus der Gruppe der ein- oder mehrfunktionellen Carbonsäureperoxyester, umfaßt und daß die Bestandteile (a) bis (f) in zwei räumlich voneinander getrennten Pasten vorliegen, wobei die Bestandteile (a), (b) und (c) in der Basispaste, der Bestandteil (d) in der Katalysatorpaste und die Bestandteile (e) und (f) in der Basis- und/oder Katalysatorpaste vorliegen.

2. Polymerisierbare Dentalmasse nach Anspruch 1,
**dadurch gekennzeichnet, daß** eine der beiden räumlich voneinander getrennten Pasten (i) ein Barbitursäurederivat und/oder ein Malonylsulfamid und (ii) ein organisches Peroxid, ausgewählt aus der Gruppe der ein- oder mehrfunktionellen Carbonsäureperoxyester, aufweist.

3. Polymerisierbare Dentalmasse nach einem der Ansprüche 1 oder 2,
**dadurch gekennzeichnet, daß** das organische Peroxid ein Carbonsäuretertiärbutyl-peroxyester ist.

4. Polymerisierbare Dentalmasse nach einem der Ansprüche 1 bis 3,
**dadurch gekennzeichnet, daß** das organische Peroxid ein Kohlensäure-tertiärbutyl-peroxy-(2-ethylhexyl)ester oder ein 3,5,5-Trimethylhexansäure-tertiärbutyl-peroxyester ist.

5. Polymerisierbare Dentalmasse nach einem der vorhergehenden Ansprüche,
**dadurch gekennzeichnet, daß** der Bestandteil (a) in einer Konzentration von 30 bis 80 Gew. %, bevorzugt in einer Konzentration von 45 bis 70 Gew. %, enthalten ist.

6. Polymerisierbare Dentalmasse nach einem der vorhergehenden Ansprüche,
**dadurch gekennzeichnet, daß** der Bestandteil (b) in einer Konzentration von 0 bis 58,99 Gew. %, insbesondere in einer Konzentration von 0 bis 33,9 Gew. %, enthalten ist.

7. Polymerisierbare Dentalmasse nach einem der vorhergehenden Ansprüche,
**dadurch gekennzeichnet, daß** der Bestandteil (c) in einer Konzentration von 0 bis 3 Gew. %, insbesondere in einer Konzentration von 0,05 bis 2 Gew. %, enthalten ist.

8. Polymerisierbare Dentalmasse nach einem der vorhergehenden Ansprüche,
**dadurch gekennzeichnet, daß** der Bestandteil (d) in einer Konzentration-von 0,01 bis 3 Gew. %, bevorzugt in einer Konzentration von 0,05 bis 2-Gew. %, enthalten ist.

9. Polymerisierbare Dentalmasse nach einem der vorhergehenden Ansprüche,
**dadurch gekennzeichnet, daß** der Bestandteil (e) in einer Konzentration von 10 bis 68,99 Gew. %, bevorzugt in einer Konzentration von 20 bis 53,9 Gew. %, enthalten ist.

10. Polymerisierbare Dentalmasse nach einem der vorhergehenden Ansprüche,
**dadurch gekennzeichnet, daß** der Bestandteil (f) in einer Konzentration von 1 bis 20 Gew. %, bevorzugt in einer Konzentration von 1 bis 15 Gew. %, enthalten ist.

11. Verwendung der polymerisierbaren Dentalmasse nach den Ansprüchen 1 bis 10 zur Herstellung von Materialien für den Einsatz als Füllungsmaterial, Stumpfaufbaumaterial, Befestigungszement, provisorisches Kronenund Brückenmaterial, zahntechnischer Werkstoff oder zur Herstellung von Inlays, Onlays, Verblendschalen, Modellmaterialien.

## Claims

1. A polymerizable dental substance, containing
(a) 10 to 98.949% by weight of at least one bi- or higher functional ethylenically unsaturated monomer,
(b) 0 to 88.999% by weight of at least one monofunctional ethylenically unsaturated monomer,
(c) 0.05 to 5% by weight of an accelerator,
(d) 0.001 to 5% by weight of a redox initiator system which can induce free-radical polymerization,
(e) 0 to 88.999% by weight of fillers, thixotropic auxiliaries, retardants and other auxiliaries and
(f) 1 to 30% by weight of a customary plasticizer,
**characterized in that** the redox initiator system comprises (i) a barbituric acid derivative and/or a malonylsulphamide and (ii) an organic peroxide, selected from the group consisting of the mono- or polyfunctional carboxylic acid peroxy esters, and **in that** the constituents (a) to (f) are present in two pastes which are spatially separate from one another, where the constituents (a), (b) and (c) are present in the base paste, the constituent (d) is present in the catalyst paste and the constituents (e) and (f) are present in the base paste and/or catalyst paste.

2. Polymerizable dental substance according to Claim 1,
**characterized in that** one of the pastes which are spatially separate from one another (i) contains a barbituric acid derivative and/or a malonylsulphamide and (ii) an organic peroxide selected from the group consisting of the mono- or polyfunctional carboxylic acid peroxy esters.

3. Polymerizable dental substance according to one of Claims 1 or 2,
**characterized in that** the organic peroxide is a carboxylic acid tertiary butyl peroxy ester.

4. Polymerizable dental substance according to one of Claims 1 to 3,
**characterized in that** the organic peroxide is a carboxylic acid tertiary butyl peroxy (2-ethyl-hexyl) ester or a 3,5,5-trimethylhexanoic acid tertiary butyl peroxy ester.

5. Polymerizable dental substance according to one of the preceding claims,
**characterized in that** the constituent (a) is contained in a concentration of 30 to 80% by weight, preferably in a concentration of 45 to 70% by weight.

6. Polymerizable dental substance according to one of the preceding claims,
**characterized in that** the constituent (b) is contained in a concentration of 0 to 58.99% by weight, in particular in a concentration of 0 to 33.9% by weight.

7. Polymerizable dental substance according to one of the preceding claims,
**characterized in that** the constituent (c) is contained in a concentration of 0 to 3% by weight, in particular in a concentration of 0.05 to 2% by weight.

8. Polymerizable dental substance according to one of the preceding claims,
**characterized in that** the constituent (d) is contained in a concentration of 0.01 to 3% by weight, preferably in a concentration of 0.05 to 2% by weight.

9. Polymerizable dental substance according to one of the preceding claims,
**characterized in that** the constituent (e) is contained in a concentration of 10 to 68.99% by weight, preferably in a concentration of 20 to 53.9% by weight.

10. Polymerizable dental substance according to one of the preceding claims,
**characterized in that** the constituent (f) is contained in a concentration of 1 to 20% by weight, preferably in a concentration of 1 to 15% by weight.

11. The use of the polymerizable dental substance according to Claims 1 to 10 for the production of materials for use as a filling material, material for building up stumps, fixative cement, temporary crown and bridge material, dental material or for the production of inlays, onlays, facing shells, model materials.

## Revendications

1. Masse dentaire polymérisable, contenant
(a) 10 à 98,949% en poids d'au moins un monomère éthyléniquement insaturé, difonctionnel ou à fonctionnalité supérieure,
(b) 0 à 88,999% en poids d'au moins un monomère éthyléniquement insaturé monofonctionnel,
(c) 0,05 à 5% en poids d'un accélérateur
(d) 0,001 à 5% en poids d'un système d'initiateur redox, qui peut déclencher la polymérisation radicalaire,
(e) 0 à 88,999% en poids de charge, d'adjuvants de thixotropie, de retardateurs et d'autres adjuvants et
(f) 1 à 30% en poids d'un plastifiant usuel
**caractérisée en ce que** le système d'initiateur redox comprend (i) un dérivé d'acide barbiturique et/ou un malonylsulfamide et (ii) un peroxyde organique, choisi dans le groupe des peroxyesters d'acides carboxyliques monofonctionnels ou polyfonctionnels et **en ce que** les constituants (a) à (f) se trouvent dans deux pâtes séparées l'une de l'autre dans l'espace, les constituants (a), (b) et (c) se trouvant dans la pâte de base, le constituant (d) dans la pâte de catalyseur et les constituants (e) et (f) dans la pâte de base et/ou la pâte de catalyseur.

2. Masse dentaire polymérisable selon la revendication 1, **caractérisée en ce qu'**une des deux pâtes séparées l'une de l'autre dans l'espace présente (i) un dérivé de l'acide barbiturique et/ou un malonylsulfamide et (ii) un peroxyde organique, choisi dans le groupe des peroxyesters d'acides carboxyliques monofonctionnels ou polyfonctionnels.

3. Masse dentaire polymérisable selon l'une quelconque des revendications 1 ou 2, **caractérisée en ce que** le peroxyde organique est un tert-butylperoxyester de l'acide carboxylique.

4. Masse dentaire polymérisable selon l'une quelconque des revendications 1 à 3, **caractérisée en ce que** le peroxyde organique est un ester tert-butyl-peroxy-2-éthylhexylique de l'acide carbonique ou un tert-butylperoxyester de l'acide 3,5,5-triméthylhexanoïque.

5. Masse dentaire polymérisable selon l'une quelconque des revendications précédentes, **caractérisée en ce que** le constituant (a) est contenu en une concentration de 30 à 80% en poids, de préférence en une concentration de 45 à 70% en poids.

6. Masse dentaire polymérisable selon l'une quelconque des revendications précédentes, **caractérisée en ce que** le constituant (b) est contenu en une concentration de 0 à 58,99% en poids, en particulier en une concentration de 0 à 33,9% en poids.

7. Masse dentaire polymérisable selon l'une quelconque des revendications précédentes, **caractérisée en ce que** le constituant (c) est contenu en une concentration de 0 à 3% en poids, en particulier en une concentration de 0,05 à 2% en poids.

8. Masse dentaire polymérisable selon l'une quelconque des revendications précédentes, **caractérisée en ce que** le constituant (d) est contenu en une concentration de 0,01 à 3% en poids, de préférence en une concentration de 0,05 à 2% en poids.

9. Masse dentaire polymérisable selon l'une quelconque des revendications précédentes, **caractérisée en ce que** le constituant (e) est contenu en une concentration de 10 à 68,99% en poids, de préférence en une concentration de 20 à 53,9% en poids.

10. Masse dentaire polymérisable selon l'une quelconque des revendications précédentes, **caractérisée en ce que** le constituant (f) est contenu en une concentration de 1 à 20% en poids, de préférence en une concentration de 1 à 15% en poids.

11. Utilisation de la masse dentaire polymérisable selon les revendications 1 à 10 pour la préparation de matériaux en vue de l'utilisation comme matériau de remplissage, matériau de construction d'un moignon, ciment de fixation, matériau provisoire pour couronne ou bridge, matériau technique dentaire ou pour la réalisation d'inlays, d'onlays, de placages ou de matériaux de moulage.
